# EUROPEAN PATENT SPECIFICATION

(11) **EP 1 257 248 B1**
(45) Date of publication and mention of the grant of the patent: **05.10.2011**
(21) Application number: 01916128.0
(22) Date of filing: 16.02.2001
(51) Int. Cl.: A61F 13/00, A01N 35/00, A61K 31/365, A61P 31/10, A61K 31/4174

(54) **PHARMACEUTICAL COMPOSITION**
PHARMAZEUTISCHE ZUSAMMENSETZUNG
COMPOSITION PHARMACEUTIQUE

(30) Priority: 16.02.2000 US 182924 P
(43) Date of publication of application: 20.11.2002
(73) Proprietor: CPEX Pharmaceuticals, Inc., Exeter, NH 03833 (US)
(72) Inventor: GYURIK, Robert, J., Exeter, NH 03833 (US)
(74) Representative: Chajmowicz, Marion
(86) International application number: PCT/US2001/005302
(87) International publication number: WO 2001/060325

(56) References cited:
- EP-A2- 0 027 286
- WO-A-96/12467
- WO-A1-99/39680
- WO-A1-99/49835
- US-A- 5 023 252
- US-A- 5 214 163
- US-A- 5 264 206
- US-A- 5 693 828
- US-A- 5 731 303
- US-B1- 6 495 124
- PRAUSNITZ M R ET AL: "Current status and future potential of transdermal drug delivery", NATURE REVIEWS DRUG DISCOVERY 200402 GB, vol. 3, no. 2, February 2004 (2004-02), pages 115-124, ISSN: 1474-1776
- DATABASE BIOSIS [Online] BIOSCIENCES INFORMATION SERVICE, PHILADELPHIA, PA, US; 1995, GOLDBERG-CETTINA MELINDA ET AL: "Enhanced transdermal delivery of estradiol in vitro using binary vehicles of isopropyl myristate and short-chain alkanols", Database accession no. PREV199598097252 & INTERNATIONAL JOURNAL OF PHARMACEUTICS (AMSTERDAM), vol. 114, no. 2, 1995, pages 237-245, ISSN: 0378-5173

## Description

### Field of the Invention

The present invention relates to a composition useful for drug delivery. More particularly, the present invention relates to a pharmaceutical composition which includes a permeation enhancer as defined in the claims, that is, a material which is capable of increasing the rate of passage of a pharmaceutical compound through a nail and/or membrane.

The present invention will be described initially with respect to its use in the treatment of fungal infections of the nail of the finger or toe. It should be understood, however, that the present invention can be used in other applications as well, as described below.

Onychomycosis is a fungal infection of the nail which affects 7 to 8 % of the North American population, including 15 to 20 % of adults aged 40 to 60 years and 25 to 40 % of adults over 60. Onychomycosis causes thickening and discoloration of the nail and can cause also loss or deterioration of the nail, pain, impaired circulation, and difficulty in walking, among other adverse effects.

Treatment of onychomycosis has in the past included measures such as removal of the infected portion of the nail or removal of the entire nail. This form of treatment, however, may lead to permanent damage to the nail and the nail, if it grows back, may grow back in a misshapen form. In addition, there is no guarantee that onychomycosis is eradicated completely by nail removal.

Instead of nail removal, it is preferable to treat onychomycosis through the use of a variety of anti-fungal agents. Traditionally, such anti-fungal agents are administered orally. Upon administration, the agent moves through the body where small amounts of the agent arrive at the target area and penetrate the nail via the nail matrix. Oral administration is disadvantageous, however, in that such administration requires a prolonged treatment of about 12 weeks for toenails and about 6 to about 8 weeks for fingernails. Such prolonged treatment increases the cost of the treatment and reduces patient compliance. In addition, oral treatment creates a risk of intoxication, gastrointestinal irritation, nausea, adverse drug-to-drug interactions, drug-induced rash, and other adverse side affects. Moreover, variable rates of absorption and metabolism are often encountered in oral treatment.

Another method of treating onychomycosis involves the topical administration of a composition containing an anti-fungal agent. Such method of treatment is herein described.

### Reported Developments

It is known to treat the fungal infection of the nail by applying to the nail a pharmaceutical composition which contains an anti-fungal agent, for example, clotrimazole. The pharmaceutical composition is typically in the form of a nail lacquer, for example, as described in U.S. Patent No. 5,264,206. Such lacquers, however, have had limited success in the treatment of onychomycosis because the anti-fungal agents are not particularly effective in penetrating the nail and the skin surrounding and underneath the nail (the "nail bed").

The addition to the pharmaceutical composition of a permeation enhancer to improve the delivery of the anti-fungal drug to the target area has been proposed. For example, U.S. Patent No. 5,346,692 discloses a nail lacquer composition which comprises an anti-fungal agent and urea as a permeation enhancer. Urea functions, however, by breaking down and disrupting the nail or skin in order to permit the anti-fungal agent to penetrate the deeper layers of the nail and nail bed. Accordingly, the use of urea results in damage to the nail and membrane.

Another composition for treating onychomycosis by applying the composition to the nail is disclosed in U.S. Patent No. 5,814,305. This composition comprises: (A) an anti-fungal agent which is at least partially soluble in water; (B) a hydrophilic permeation enhancer; (C) water; and (D) alcohol. Anti-fungal agents of the allylamine family, such as terbinafine hydrochloride and naftifine hydrochloride, are preferred for use in the compositions of the '305 patent. The patent discloses the use of the following hydrophilic permeation enhancers: glycols; glycol diethers; caprolactam; dimethylisosorbide; isopropylidene glycerol; dimethylimidazolidinone; N-methyl-pyrrolidone-2; pyrrolidone-2; ethyl lactate; and polyoxyethylenated glycerides. A disadvantage associated with the use of the composition described in the '305 patent is that a hydrophilic delivery system is used and most anti-fungal agents, in particular the azoles, are lipophilic. Lipophilic enhancers have been shown to be more efficient than hydrophilic enhancers in increasing the absorption of pharmaceutical compounds into the strata of the membrane and the nail. Moreover, lipophilic antifungal agents are preferred since they have a tendency to penetrate more readily the membrane.

WO 99/39680 discloses various lacquers comprising anti-fungal agents, membrane-compatible permeation enhancers and polymeric film-forming agents, without disclosing the membrane-compatible permeation enhancers of formula I used herein. The present invention provides an improved composition that can be applied to the nail for the purpose of treating a fungal infection that afflicts the nail and or nail bed and that can be used in other applications also.

### Summary of the Invention

It is herein described a composition comprising: (A) a pharmaceutical compound; (B) a membrane-compatible permeation enhancer; and (C) a polymeric film-forming agent.

More particulary, the present invention provides a composition which is particularly suited to treating a nail disorder and which comprises a hydrocarbon-based liquid nail lacquer comprising a solution of: (A) an anti-fungal agent in a pharmaceutically-effective amount; (B) a membrane-compatible permeation enhancer in an amount effective to enhance penetration through a membrane or nail of said anti-fungal agent; (C) a polymeric film-forming agent in an amount effective to form an adherent polymeric film on a membrane or nail; and (D) solvent for forming said solution, wherein the permeation enhancer is a compound of formula I as described below.

It is further described a method for administering to the body through the nail or membrane of the body comprising: (A) applying to the nail or membrane a liquid composition comprising a pharmaceutical compound, such membrane-compatible permeation enhancer, and a polymeric film-forming agent; (B) forming from said liquid composition a solid film which adheres to the nail or membrane; and (C) maintaining said film on the nail or membrane for a period of time sufficient for delivery of the pharmaceutical compound to the body through the nail or membrane.

It is also described a method for forming a solid adherent film comprising a pharmaceutical compound and an enhancer therefore comprising: (a) providing a solution in which the pharmaceutical compound, the enhancer, and a polymeric film-forming agent are dissolved, the solution containing a co-solvent which includes: (i) a highly volatile solvent for the enhancer in which the enhancer is highly soluble; and (ii) a less volatile solvent in which the enhancer is less soluble; and (b) applying said soluble solution to the nail or membrane; (c) forming from said solution a solid film which includes the pharmaceutical compound and the enhancer by permitting the highly volatile solvent to evaporate at a faster rate than the solvent having lower volatility; and (d) drying the resulting film.

It will be appreciated from the discussion which follows that the present invention provides an important advantage in that it can be used effectively to treat body conditions such as onychomycosis or other fungal infections, bacterial infections, and inflammation.

### Detailed Description of the Invention

The composition of the present invention comprises a pharmaceutically effective amount of an anti-fungal agent.

More particularly, the composition comprises a pharmaceutically effective amount of an anti-fungal agent which is capable of treating an infection residing in the nail and/or the nail bed. Essentially, any suitable anti-fungal agent may be employed. The anti-fungal agent may be present also in combination with an anti-inflammatory agent and/or another anti-microbial agent such as, for example, an anti-bacterial agent or an anti-viral agent.

It is known that anti-fungal agents can function in various ways. For example, one class of anti-fungal agents works by impairing the functions of membrane-bound enzyme systems in the fungal cell membrane. Examples of such anti-fungal agents are azoles, for example, tioconazole, econazole, miconazole, terconazole; clotrimazole, bifonazole, butaconazole, chlordantoin, chlormidazole, cloconazole, enilconazole, fenticonazole, isoconazole, ketoconazole, omoconazole, oxiconazole nitrate, and sulconazole.

It is known also that another class of anti-fungal agents functions by inhibition of oxidosqualene cyclase, thus deterring or preventing the formation of the requisite ergosterol. Examples of such agents are allylamines, for example, terbinafine and naftifine.

Still another class of anti-fungal agents functions by compromising the integrity of the fungal cell membrane; an example of such an anti-fungal agent is ciclopirox.

Anti-fungal agents that work by varied modes of action as fungicidal or fungistatic agents may be employed also, for example, amorolfine, griseofluvin, nystatin, amphotericin B.

Clotrimazole and fluconazole are preferred anti-fungal agents for use in the practice of the present invention.

The pharmaceutical compound (i.e. the anti-fungal agent) is present in the composition in a pharmaceutically effective concentration, which may be determined by those of ordinary skill in the art. Preferably, the concentration does not exceed the maximum amount that remains soluble in the composition, a parameter which can be determined readily also. For guideline purposes, it is believed most applications will involve the use of the pharmaceutical compound in an amount of about 0.1% to about 15% by weight of the composition. A preferred amount of the compound is about 1% to about 10% by weight of the composition, more preferably about 1% to about 8% by weight of the composition, and most preferably about 2% to about 5% by weight of the composition.

The composition comprises also a membrane-compatible permeation enhancer which is capable of increasing the rate of passage of the pharmaceutical compound through a nail and/or membrane, that is, a layer of body tissue, for example, skin. The term "membrane-compatible permeation enhancer" means a compound which increases the rate of delivery of the pharmaceutical compound through the nail/membrane without damage.

The membrane-compatible enhancer of the present invention can be applied safely to the membrane or to a nail without causing damage to the membrane or nail.

The membrane-compatible permeation enhancers used herein are compounds which are described in U.S. Patent No. 5,023,252 to Hsieh (assigned to the same assignee as the present invention) and which have the structure of Formula I. 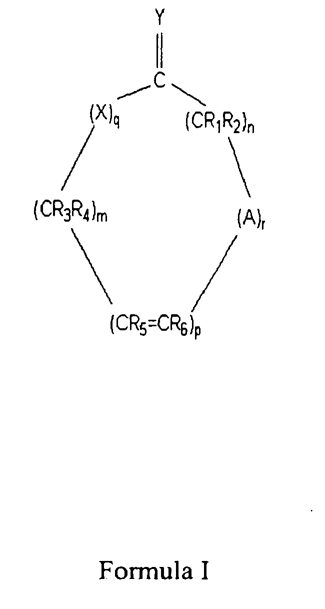 wherein X and Y are oxygen, sulfur or an imino group of the structure 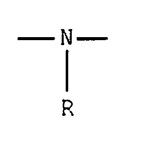 or =N-R, with the proviso that when Y is an imino group, X is an imino group, and when Y is sulfur, X is sulfur or an imino group, A is a group having the structure 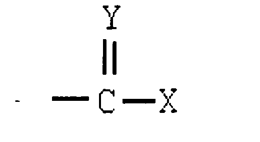 wherein X and Y are as defined above, m and n are integers having a value from 1 to 20 and the sum of m+n is not greater than 25, p is an integer having a value of 0 or 1, q is an integer having a value of 0 or 1, r is an integer having a value of 0 or I, and each of R, R₁, R₂, R₃, R₄, R₅, and R₆ is independently hydrogen or an alkyl group having from 1 to 6 carbon atoms which may be straight chained or branched, provided that only one of R₁ to R₆ can be alkyl group, with the proviso that when p, q and r are 0 and Y is oxygen, then m+n is at least 11, and with the further proviso that when X is an imino group, q is equal to 1, Y is oxygen, and p and r are 0, then m+n is at least 11. For convenience, an enhancer of Formula 1 above is referred to herein as a "macrocyclic enhancer".

Macrocyclic enhancers have several desirable properties in addition to their "enhancing" properties. For example, they have the ability to form a stable homogenous solution with the other components of the composition of the present invention and to function as a plasticizer and fluidizer in a film which is formed from the composition. This improves the performance properties of the film by rendering it pliable. This is surprising, because the preferred enhancer compounds, for example, oxacyclobexadecan-2-one, are solids at room temperature. However, a liquid composition of the present invention in which the enhancer is dissolved is capable of forming a solid polymeric film which is clear and elastic. In addition, the film is capable of being resolubilized by successive applications of the film, providing a continuous, replenished delivery system without discontinuity or disruption of the pharmaceutical compound.

Those skilled in the art will recognize which permeation enhancers are used preferably with the particular pharmaceutical compounds that are included in the composition. Particularly preferred membrane-compatible permeation enhancer for use in the practice of the present invention are oxacyclohexadecan-2-one, muscone, civetone, and normuscone.

The membrane-compatible permeation enhancer is present in the composition in a concentration effective to enhance penetration through the nail and/or membrane of the pharmaceutical compound to be delivered. The "effective amount" can be determined readily by the skilled artisan. For guideline purposes, it is believed most applications will involve the use of the membrane-compatible permeation enhancer in an amount of about 1% to about 25% by weight of the composition, preferably about 5% to about 20% by weight of the composition.

The composition comprises also a polymeric film-forming agent or mixture of film-forming agents. Essentially, there can be used any polymer that is capable of forming a film from which the pharmaceutical compound can be delivered to the nail or membrane, for example, the nail bed. For example, occlusive and semi-occlusive polymers which are known for use in transdermal drug delivery can be used. Because the primary objective is drug delivery, even those polymers otherwise rejected for cosmetic end uses for less than ideal cosmetic properties can be used in the practice of the present invention.

Examples of suitable polymeric film-forming agents include polymers of acrylic acid, acrylic acid esters and copolymers thereof; polymers of methacrylic acid, methacrylic acid esters and copolymers thereof; polymers of vinyl acetate and copolymers thereof with acrylic acid and acrylic acid esters; copolymers of methyl vinyl ethers with maleic acid, maleic acid alkyl esters, and combinations thereof; copolymers of vinyl pyrrolidone with styrene; poly(vinyl butyrates); polymeric cellulose derivatives such as cellulose acetate phthalate, cellulose acetate butyrate, cellulose acetate propionate, cellulose nitrate, cellulose sulfate, ethyl cellulose, and cellulose acetate; terpolymers of vinyl acetate with butyl maleate and isobornyl acetate; terpolymers of vinyl caprolactam with vinyl pyrrolidone and dimethylamino ethyl methacrylate. The film-forming agent can be used in solid, for example, powdery form in formulating a composition of the present invention. In addition, the composition may be formulated also by use of a latex.

Examples of preferred polymeric film-forming agents include: quaternary ammonium-containing acrylic acid ester; methacrylic acid ester copolymers known as ammonio methacrylate copolymers such as, for example, ethyl acrylate-[2-(methacryloyloxy) ethyl] trimethylammonium chloride-methyl methacrylate copolymer; and substituted copolymers of alkylated poly(vinyl pyrrolidones). These polymeric film-forming agents are preferred because they demonstrate superior adhesive, water- resistance and hardness properties. Particularly preferred are polymeric film-forming agents that have been registered with regulatory agencies for pharmaceutical use, including, but not restricted to the European or United States Pharmacopeia, and the Japanese Pharmaceutical Excipients Compendia.

The polymeric film-forming agent is present in an amount effective to form on the membrane or the nail an adherent polymeric film. Amounts can be determined readily for any particular application. For guideline purposes, it is believed most applications will involve the use of the polymeric film-forming agent in an amount of about 0.1 % to about 35%, preferably about 5% to about 35% by weight of the composition and more typically, and most preferably, in an amount of about 10% to about 25% by weight of the composition.

The composition comprises also a solvent for those ingredients to be liquified. Essentially any solvent or solvent combination which is a suitable vehicle for the composition of the present invention can be employed. Examples of such solvents are alcohols, esters, ethers, aromatic hydrocarbons, aldehydes, ketones, mono-, di- and tri-glycerides, and the like.

It is believed that the solvents that will be used most widely in the practice of the present invention are ethanol, ethyl acetate, butyl acetate, isopropanol, acetone, methyl ethyl ketone, triacetin, tripropionin, diethylene glycol monoethyl ether, and isopropyl acetate, and a mixture of two or more of the aforementioned. Ethanol, ethyl acetate, butyl acetate, isopropanol, methyl ethyl ketone and acetone are preferred solvents as they evaporate and dry readily when applied.

The solvent is present in the composition in an amount sufficient to solubilize the ingredients that are to be liquified, for example, a solid polymeric film-forming agent, and other ingredients, without resulting in unsatisfactory drying times or film properties. For guideline purposes, it is believed most applications will involve the use of the solvent in an amount of about 30% to about 80% by weight of the composition and more typically, and preferably, in an amount of about 40% to about 70% by weight of the composition.

In forming preferred compositions within the scope of the present invention, there are several factors that should be taken in to account in selecting the particular pharmaceutical compound, enhancer, film-forming agent, and solvent that comprises the composition. The preferred composition is one which is capable of forming a solid film in a manner such that the concentrations of the pharmaceutical compound and enhancer are relatively high in the portion of the solid film which is contiguous to the surface on which the solid film is formed, for example, a nail surface.

By way of background, it is noted that the term "fugacity" is used to refer to the measure of the escaping tendency of a solute in a solution and that the fugacity of a solute follows Henry's Law for ideal states. Various methods can be used in order to increase the fugacity of the pharmaceutical compound and enhancer, that is, to increase the concentrations thereof at the desired site.

For example, with respect to a pharmaceutical compound which is basic in nature, the polymeric film-forming agent used in combination therewith is preferably also basic in nature. Such basic compounds tend to repel each because of the positive charges associated therewith, thus increasing the fugacity of the pharmaceutical compound. Examples of polymeric film-forming agents that have a basic moiety or functionality are acrylate copolymers, for example, those that have intermittently distributed dimethylamino functionalities. Another method that can be used to increase the fugacity of the pharmaceutical compound and the film-forming agent is to add to the solution thereof a basic compound, for example, TRIS amino or triethanolamine.

With respect to the use of an acidic-type pharmaceutical compound, for example, an acidic anti-fungal agent such as ciclopirox, there can be used a polymeric film-forming agent that is acidic in nature. Examples of film-forming agents that have acid functionality are polymers or co-polymers of acrylic acid or methacrylic acid, for example, co-polymers of such acids with esters thereof.

The following is a description of a method to increase the fugacity of the permeation enhancer. The method involves formulating a composition comprising a permeation enhancer dissolved in a volatile solvent, for example, a solvent which has a vapor pressure sufficient to allow it to evaporate within 5 minutes after application, and a less volatile solvent in which the permeation enhancer has limited solubility, for example, a solubility of up to 5 weight percent. Such a composition comprises co-solvents for the permeation enhancer. In the formation of the film from the composition, the volatile solvent evaporates preferentially relative to the less-volatile solvent which remains (but temporarily) in the composition. As this occurs, the fugacity of the permeation enhancer increases. A cosolvent that increases the fugacity of a permeation enhancer upon the evaporation of a more volatile solvent in which the permeation enhancer is highly soluble includes, for example, propylene glycol.

An exemplary method for increasing the fugacity of both the pharmaceutical compound for example an anti-fungal agent, which has limited solubility in water, and a permeation enhancer which has relatively high hydrophobicity (more limited water solubility then the pharmaceutical compound) is to include water in the composition, for example, in an amount up to about 20% by weight of the composition, preferably about 1% to about 10%, and most preferably about 3% to about 7% by weight of the composition.

The foregoing description provides exemplary methods which may be employed to maximize the release of both the pharmaceutical compound and the enhancer or to provide more subtle increases and decreases in the fugacity of each component together or independently. Accordingly, the present invention provides a means by which the release rate of either or both of the aforementioned components may be designed for more prolonged, controlled, or direct release of the medication.

One or more plasticizers can be included in the composition to impart desired properties to the film formed from the composition. In selecting a plasticizer and amount to use, there should be taken into account whether the permeation enhancer that is present in the composition has plasticizing properties. Essentially any plasticizer can be employed. Examples of plasticizers are propylene glycol, diethylene glycol monoethyl ether, propylene glycol monopropyl ether, polyethylene and poly(propylene glycol), triacetin, tripropionin, castor oil, camphor, phthalates, particularly dibutyl phthalate and diethyl phthalate, benzyl alcohol, phenethyl alcohol, and N-methyl-2-pyrrolidone, and a mixture of two or more of the aforementioned. As is known in the art, the plasticizer should be matched with the polymeric film-forming agent that is used in the composition.

It is believed that the plasticizers that will be used most widely in the practice of the present invention will be propylene glycol, diethylene glycol monoethyl ether, polyethylene and poly(propylene glycol), triacetin, and tripropionin, and a mixture of two or more of the aforementioned. Propylene glycol, is among the preferred plasticizers.

The plasticizer is present in an amount sufficient to provide the desired plasticizing properties to the polymeric film that is formed from the composition. For guideline purposes, it is believed most applications will involve the use of the plasticizer in an amount of 1% to about 25% by weight of the composition and more typically in an amount of about 1% to about 10% by weight of the composition.

The composition may include also other art-recognized components in art-recognized quantities. A coloring agent may be used, for example, a dye, color pigment, color lake, pearl gloss dye or pigment, for example, titanium dioxide, and the like. Other components include colloid stabilizers, UV stabilizers, antibacterial or bacteriostatic substances such as quaternary ammonium antimicrobial agents, for example, cetyl pyridinium chloride, and benzalkonium chloride, anti-oxidants, for example, BHA, BHT, parabens, vitamin E and its derivatives, anti-microbial chelating agents, for example, EDTA and citric acid, and neutralizing agents, for example, TRIS amino, triethylamine, triethanolamine, 2-methyl-2-amino-1-propanol, citric acid, and sorbic acid.

The composition of the present invention is applied to the membrane or the nail like any conventional composition that is capable of forming a solid film. The film can be formed from multiple coats of the composition. One or more successive coats may be applied typically after the underlying film is formed as the solvent evaporates and the wet film dries. Periodic replacement of the film may be required to maintain the desired drug dosage regimen.

It is preferred that the components of the liquid composition be compatible with each other so that a clear film, free from clouding before and after application results. The film should remain preferably clear and non-cloudy for each successive application. Clearness of the film is an indication that the desired molecules of anti-fungal agent and permeation enhancer are in a glassy state, and have not crystallized; otherwise the molecules may cease to migrate and not reach the intended target, the infected nail and membrane. Such a "cold flow" condition is important to remediating the infection in an efficient manner.

As can be appreciated from the discussion above, the present invention provides an important advantage in that it can be used effectively to treat body conditions such as onychomycosis or other fungal infections, bacterial infections, and inflammation.

### EXAMPLES

Examples below are illustrative of compositions of the present invention. The concentrations of the ingredients comprising the compositions are given in percent by weight relative to the total weight of composition.

Example Nos. 1 to 5 are examples of anti-fungal nail lacquers of the present invention. In the following examples, the polymeric film-forming agent, in powder or pelletized form, was dissolved in solvent while mechanically stirring at room temperature. After the film-forming agent was dissolved in the solvent, the pharmaceutical compound and the permeation enhancer, each in solid form, were added with stirring. After the pharmaceutical compound and the permeation enhancer were dissolved, the plasticizer and water were added. The entire mixture was then stirred until homogenous.

### Example No. 1

| | Wt. % |
|---|---|
| clotrimazole, USP (Sifavitor) | 4 % |
| Eudragit RL 100 powder (Röhm) film-forming agent (ethyl acrylate, methyl methacrylate, trimethylammonioethyl methacrylate copolymer) | 15 % |
| oxacyclohexadecan-2-one (Firmenich) - permeation enhancer | 15 % |
| propylene glycol, USP - plasticizer | 5 % |
| ethanol, USP - solvent (200 proof) | 58 % |
| water | 3 % |

Six (6) human patients with onychomycosis of the big toe, with at least 33% of the surface of the nail infected, were each treated once daily in the evening with approximately 20-30 mg of a nail lacquer having the formulation of Example No. 1. The treatment was applied each day for seven days. The lacquer was removed on the seventh day of each week cycle by dissolving with 70% isopropyl alcohol. Thereafter, the cycle was repeated for 120 days. After 60 days, substantial improvements were noted in all cases. Nails in all cases became clearer, harder, and the discoloration disappeared gradually. The cure proceeded from the cuticle to the distal nail. Complete cures, indicated by completely normal appearing nails, were achieved in all cases within 120 days of the beginning of the treatment. In two of the patients who were experiencing pain and tenderness in the nail, surprisingly, within one month after beginning treatment, the pain and tenderness subsided.

### Example No. 2

| | Wt. % |
|---|---|
| fluconazole (Quimica Sintética, SA) | 2 % |
| PVP/VA S-630 (ISP) powder film-forming agent (vinylpyrrolidone/vinyl acetate copolymer) | 18% |
| oxacyclohexadecan-2-one (Firmenich) - permeation enhancer | 14 % |
| propylene glycol - plasticizer | 4% |
| ethanol, USP - solvent (200 proof) | 57% |
| tripropionin - plasticizer | 1 % |
| methyl-2-amino-1-propanol - neutralizing-agent | 1% |
| water | 3 % |

### Example No. 3

| | Wt.% |
|---|---|
| clotrimazole (Sifavitor) | 4 % |
| Eudragit RL PO (Röhm) pellets - film-forming agent (ethyl acrylate, methyl methacrylate, tri-methylammonioethyl methacrylate copolymer) | 15% |
| oxacyclohexadecan-2-one (Firmenich) - permeation enhancer | 15% |
| propylene glycol, USP - plasticizer | 5 % |
| ethanol, USP - solvent (200 proof) | 58% |
| water | 3 % |

### Example No. 4

| | Wt. % |
|---|---|
| terconazole (Quimica Sintética, SA) | 4% |
| Gantrez® MS-955 (ISP) powder - film-forming agent (2-butenedioic acid monobutyl ester, methoxyethylene copolymer) | 15% . |
| cyclopentadecanone (Firmenich) - permeation enhancer | 5% |
| polyethylene glycol (MW 400) - plasticizer | 5 % |
| ethanol, USP - solvent (200 proof) | 58 % |
| water | 3 % |

### Example 5

| | Wt. % |
|---|---|
| fluconazole (Quimica Sintética, SA) | 5% |
| Poviderm™ SK3 (ISP) powder - film-forming agent (2-pyrrolidone, 1-ethenyl-, homopolymer) | 15% |
| Oxacyclohexadecan-2-one (Firmenich) - permeation enhancer | 15% |
| polyethylene glycol (USP) - plasticizer | 10 % |
| ethanol, USP (190 proof) - solvent | 55 % |
| water | 3% |

The next example is illustrative of a composition of the present invention in gel form.

### Example No. 6

| | Wt. % |
|---|---|
| clotrimazole | 1% |
| Carbopol 980 NF (BF Goodrich) powder - film-forming agent and thickening agent | 3% |
| oxacyclohexadecan-2-one (Firmenich) - permeation enhancer | 4% |
| propylene glycol - plasticizer | 5 % |
| glycerin - plasticizer and humectant | 3% |
| ethanol, USP - solvent (200 proof) | 66 % |
| triethanolamine, NF - neutralizing agent | Qs. to pH 5.5 |
| water, reagent grade | 17 % |

The gel of Example No. 6 was applied to excised guinea pig skin in a standard Franz cell apparatus (in vitro) or as a film on a 10 cm² area on live guinea pig skin (30 mg formulation applied) that had been prepared previously by shaving (in vivo). *In vitro* permeation time varied among 24, 48, and 72 hours and the total skin section was assayed. *In vivo* permeation time was seven (7) hours, after which the animals were euthanized, the skin washed, and skin separated into epidermis and dermis. The clotrimazole levels were measured in all cases by high performance liquid chromatography (HPLC). Lotrimin cream (1% clotrimazole) served as the control. The test results showed clearly a great magnitude of increase of permeation through the skin either *in vitro* or *in vivo* of the antifungal agent, clotrimazole, by one of the enhancing agents of the present invention, oxacyclohexadecan-2-one. It is believed that the present invention provides improved means for efficient and effective delivery of a pharmaceutical compound to the body by delivery thereof through a nail or membrane of the body.

## Claims

1. A hydrocarbon-based liquid nail lacquer comprising a solution of : (A) an anti-fungal agent in a pharmaceutically-effective amount ; (B) a membrane- compatible permeation enhancer in an amount effective to enhance penetration through a membrane or nail of said anti-fungal agent; (C) a polymeric film-forming agent in an amount effective to form an adherent polymeric film on a membrane or nail; and (D) solvent for forming said solution, wherein said membrane-compatible permeation enhancer is a compound of formula I 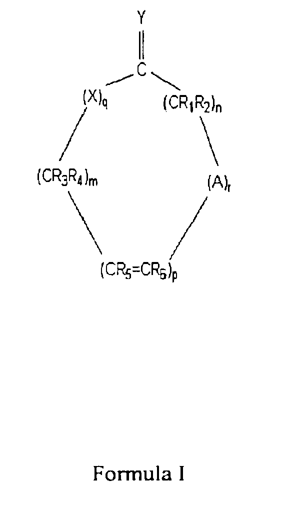 wherein X and Y are oxygen, sulfur or an imino group of the structure 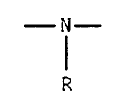 or =N-R, with the proviso that when Y is an imino group, X is an imino group, and when Y is sulfur, X is sulfur or an imino group, A is a group having the structure 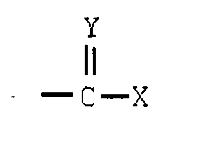 wherein X and Y are as defined above, m and n are integers having a value from 1 to 20 and the sum of m+n is not greater than 25, p is an integer having a value of 0 or 1, q is an integer having a value of 0 or 1, r is an integer having a value of 0 or 1, and each of R, R₁, R₂, R₃, R₄, R₅, and R₆ is independently hydrogen or an alkyl group having from 1 to 6 carbon atoms which may be straight chained or branched, provided that only one of R₁ to R₆ can be alkyl group, with the proviso that when p,
q and r are 0 and Y is oxygen, then m+n is at least 11, and with the further proviso that when X is an imino group, q is equal to 1, Y is oxygen, and p and r are 0, then m+n is at least 11.

2. The lacquer according to claim 1, wherein said membrane-compatible permeation enhancer is oxacyclohexadecan-2-one.

3. The lacquer according to claim 1, wherein said anti-fungal agent is an azole.

4. The lacquer of claim 1, wherein said anti-fungal agent is clotrimazole.

5. The lacquer according to claim 1, wherein said polymeric film-forming agent is selected from the group consisting of an ammoniomethacrylate copolymer; and a substituted copolymer of an alkylated poly(vinyl pyrrolidone).

6. The lacquer according to claim 1, further comprising an anti inflammatory compound.

7. The lacquer according to claim 1, wherein the solvent is selected from the group consisting of ethanol ; ethyl acetate; butyl acetate; isopropanol; acetone; methyl ethyl ketone; triacetin; tripropionin ; diethylene glycol monoethyl ether; and isopropyl acetate; and a mixture of two or more of said solvents.

8. The lacquer according to claim 1 further comprising a plasticizer.

9. The lacquer according to claim 1 including 0.1% to 15% by weight of said anti-fungal agent;
1% to 25% by weight of said enhancer; 0.1% to 35% by weight of said film-forming agent;
30% to 80% by weight of said solvent; and a plasticizer in an amount of 1% to 25% by weight.

10. A lacquer as defined in claim 1, for use in treating a fungal infection that afflicts the nail or nail bed, by application to the nail.

## Patentansprüche

1. Ein auf Kohlenwasserstoff basierender Nagellack enthaltend eine Lösung von: (A) ein anti-Pilz Mittel in einer pharmazeutisch wirksamen Menge; (B) ein Membrankompatibler Permeationsverstärker in einer Menge, die wirksam ist, um die Eindringung durch eine Membran oder Nagel dieses anti-Pilz Mittels zu verstärken; (C) ein polymeres Film-bildendes Mittel in einer Menge, die wirksam ist, um einen adhärenten polymeren Film auf einer Membran oder Nagel zu bilden; und (D) Lösungsmittel zum Bilden dieser Lösung, wobei diese Membran-kompatiblen Permeationsverstärker eine Zusammensetzung nach der Formel I ist 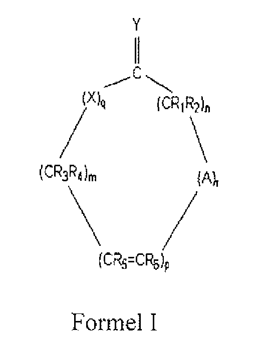 wobei X und Y Sauerstoff, Schwefel oder eine Iminogruppe der Struktur 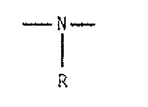 sind oder=N-R, mit der Maßgabe, dass wenn Y eine Iminogruppe ist, X eine Iminogruppe ist, und wenn Y Schwefel ist, X Schwefel oder eine Iminogruppe ist, A eine Gruppe mit der Struktur 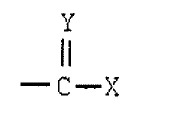 ist, wobei X und Y wie oben definiert sind, m und n ganze Zahlen sind mit einem Wert von 1 bis 20 und die Summe von m+n nicht größer als 25 ist, p eine ganze Zahl mit einem Wert von 0 oder 1 ist, q eine ganze Zahl mit einem Wert von 0 oder 1 ist, r eine ganze Zahl mit einem Wert von 0 oder 1 ist, und jede der R, R₁, R₂, R₃, R₄, R₅ und R₆ unabhängig voneinander Wasserstoff oder ein Alkylgruppe ist mit 1 bis 6 Kohlenstoffatomen, die geradekettig oder verzweigt sein können, vorausgesetzt, dass nur eine der R₁ bis R₆ eine Alkylgruppe sein kann, mit der Maßgabe, dass wenn p, q und r 0 sind und Y Sauerstoff ist, dann ist m+n mindestens 11. und mit der weiteren Maßgabe, dass wenn X eine Iminogruppe ist, q gleich 1 ist, Y Sauerstoff ist und p und r 0 sind, dann ist m+n mindestens 11.

2. Der Lack nach Anspruch 1, wobei der Membran-kompatible Permeationsverstärker Oxacyclohexadecan-2-on ist.

3. Der Lack nach Anspruch 1, wobei das anti-Pilz Mittel ein Azol ist.

4. Der Lack nach Anspruch 1, wobei das anti-Pilz Mittel Clotrimazol ist.

5. Der Lack nach Anspruch 1, wobei das polymere Film-bildende Mittel ausgewählt ist aus der Gruppe bestehend aus einem Ammoniummethacrylate-Copolymer; und einem substituierten Copolymer eines alkylierten Polyvinylpyrrolidon.

6. Der Lack nach Anspruch 1, weiter enthaltend eine anti-inflamnatorische Zusammensetzung.

7. Der Lack nach Anspruch 1, wobei das Lösungsmittel ausgewählt ist aus der Gruppe bestehend aus Ethanol; Ethylacetat, Butylacetat; Isopropanol; Aceton; Methylethylketon; Triacetin; Tripropionin; Diethylenglykolmonoethylether; und Isopropylacetat; und einer Mischung von zwei oder mehrerer dieser Lösungsmittel.

8. Der Lack nach Anspruch 1 weiter enthaltend einen Weichmacher.

9. Der Lack nach Anspruch 1 beinhaltend 0,1 Gewichts-% bis 15 Gewichts-% des anti-Pilz Mittels; 1 Gewichts-% bis 25 Gewichts-% des Verstärkers; 0,1 Gewichts-% bis 35 Gewichts-% des Film-bildenden Mittels; 30 Gewicht-% bis 80 Gewichts-% des Lösungsmittels; und einen Weichmacher in einer Menge von 1 Gewichts-% bis 25 Gewichts-%.

10. Der Lack wie in Anspruch 1 definiert zur Verwendung in der Behandlung einer Pilzinfektion, die den Nagel oder das Nagelbett betrifft, durch Anwendung an dem Nagel.

## Revendications

1. Vernis à ongles liquide à base d'hydrocarbure comprenant une solution de : (A) un agent antifongique en une quantité pharmaceutiquement efficace ; (B) un agent facilitant la perméation, compatible avec la membrane, en une quantité efficace pour accroitre la pénétration, à travers une membrane ou un ongle, dudit agent antifongique ; (C) un agent permettant la formation d'un film polymérique en une quantité efficace pour former un film polymérique adhérent sur une membrane ou un ongle ; et (D) un solvant pour former ladite solution, ledit agent facilitant la perméation, compatible avec la membrane, étant un composé de formule I 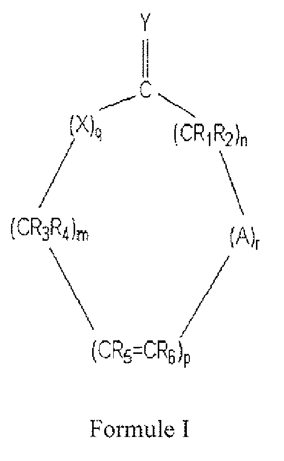 X et Y étant un atome d'oxygène, un soufre ou un groupe imino de structure 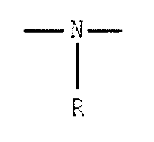 ou =N-R, à condition que quand Y est un groupe imino, X est un groupe imino, et quand Y est un soufre, X est un soufre ou un groupe imino, A est un groupe de structure 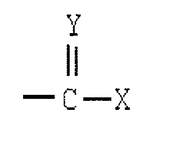 X et Y étant tels que définis ci-dessus, m et n étant des entiers compris entre 1 et 20 et la somme de m+n n'étant pas supérieure à 25, p étant un entier égal à 0 ou 1, q étant un entier égal à 0 ou 1, r étant un entier égal à 0 ou 1, et chacun des R, R₁, R₂, R₃, R₄, R₅ et R₆ étant indépendamment un atome d'hydrogène ou un groupe alkyle ayant 1 à 6 atomes de carbone lequel peut être à chaine droite ou ramifiée, sous réserve que seul un des R₁ à R₆ soit un groupe alkyle, à condition que quand p, q et r sont 0 et Y est un atome d'oxygène, alors m+n est au moins égal à 11, et à condition que, d'autre part, quand X est un groupe imino, q est égal à 1, Y est un atome d'oxygène, et p et r sont 0, alors m+n est au moins égal à 11.

2. Vernis selon la revendication 1, ledit agent facilitant la perméation, compatible avec la membrane, étant l'oxacyclohexadecan-2-one,

3. Vernis selon la revendication 1, ledit agent antifongique étant un azole.

4. Vernis selon la revendication 1, ledit agent antifongique étant du clotrimazole.

5. Vernis selon la revendication 1, ledit agent permettant la formation d'un film polymérique étant choisi dans le groupe consistant en un copolymère d'ammoniométhacrylate ; et un copolymère substitué d'une poly(vinylpyrrolidone) alkylée.

6. Vernis selon la revendication 1, comprenant, en outre, un composé anti inflammatoire.

7. Vernis selon la revendication 1, le solvant étant choisi dans le groupe consistant en éthanol, acétate d'éthyle, acétate de butyle, isopropanol, acétone, méthyl éthyl cétone, triacétine, tripropionine, diéthylène glycol monoéthyl éther, et acétate d'isopropyle, et un mélange de deux ou plus desdits solvants.

8. Vernis selon la revendication 1, comprenant, en outre, un agent plastifiant.

9. Vernis selon la revendication 1, comprenant 0,1% à 15% du poids dudit agent anti fongique ; 1% à 25% du poids dudit agent facilitant ; 0,1% à 35% du poids dudit agent formant un film ; 30% à 80% du poids dudit solvant ; et un agent plastifiant en une quantité de 1% à 25% du poids.

10. Vernis tel que défini à la revendication 1, pour une utilisation dans le traitement d'une infection fongique qui touche l'ongle ou le lit de l'ongle, par application sur l'ongle.
